# NEUE EUROPÄISCHE PATENTSCHRIFT

(11) **EP 0 350 635 B2**
(45) Veröffentlichungstag und Bekanntmachung des Hinweises auf die Entscheidung über den Einspruch: **17.07.2002**
(45) Hinweis auf die Patenterteilung: 29.09.1993
(21) Anmeldenummer: 89110743.5
(22) Anmeldetag: 14.06.1989
(51) Int. Cl.: C07C 53/08, C07C 53/12, C07C 51/12, C07C 51/56

(54) **Verfahren zur Herstellung von Essigsäure und Essigsäureanhydrid**
Process for the preparation of acetic acid and acetic anhydride
Procédé de préparation de l'acide acétique et de l'anhydride acétique

(30) Priorität: 13.07.1988 DE 3823645
(43) Veröffentlichungstag der Anmeldung: 17.01.1990
(73) Patentinhaber: Celanese Chemicals Europe GmbH, 60439 Frankfurt am Main (DE)
(72) Erfinder: Erpenbach, Heinz, Dr., D-5000 Köln (DE); Gehrmann, Klaus, Dr., D-5042 Erftstadt (DE); Jägers, Erhard, Dr., D-5303 Bornheim (DE); Kohl, Georg, D-5030 Hürth (DE)

(56) Entgegenhaltungen:
- EP-A- 0 087 869
- EP-A- 0 170 965
- EP-A- 0 173 170
- EP-A- 0 217 191
- AU-A- 4 588 789
- CA-A- 1 214 472
- DE-A- 2 450 965
- DE-A- 2 836 084
- DE-A- 2 939 839
- DE-C- 921 938
- GB-A- 906 008
- JP-A- 61 058 803
- US-A- 4 549 937
- US-A- 4 615 806

## Beschreibung

Die Erfindung betrifft ein Verfahren zur Herstellung von Essigsäure und Essigsäureanhydrid durch Umsetzung von Methanol und Methylacetat mit Kohlenmonoxid.

Essigsäure und Essigsäureanhydrid sind bedeutende aliphatische Zwischenprodukte. Die überwiegende Menge wird zur Herstellurig von Vinylacetat bzw. Celluloseacetat verwendet.

Ein Verfahren zur gemeinsamen Herstellung von Carbonsäuren und Carbonsäureanhydriden sowie ggf. Carbonsäureestern ist bereits aus der EP-A-17o 965 bekannt. Es werden dort Dialkylether mit Alkoholen unter wasserfreien Bedingungen mit Kohlenmonoxid und ggf. Wasserstoff in Gegenwart eines aus Carbonylkomplexen von Edelmetallen der Gruppe VIII des Periodensystems; einem Alkali-, Organophosphonium- oder Organoammoniumjodid; einem Alkyljodid; und ggf. aus Verbindungen carbonylbildender Nichtedelmetalle bestehenden Katalysatorsystems bei Temperaturen von 5o bis 25o°C und Drücken von 0,1 bis 120 bar umgesetzt.

Ein Verfahren zur gemeinsamen Herstellung von Essigsäure und Essigsäureanhydrid wird in der EP-A-87 869 beschrieben. Danach wird Methylacetat oder Dimethylether, Wasser und ggf. Methanol mit Kohlenmonoxid in Gegenwart eines Katalysators, bestehend aus einem Edelmetall der Gruppe VIII des Periodensystems der Elemente, einem Brom- oder Jodpromotor und einem Copromotor aus einer LewisBase oder einem Nichtedelmetall, zu einem Gemisch aus Essigsäure und Essigsäureanhydrid umgesetzt, wobei der Wassergehalt der Einsatzmischung mindestens 5,5 Gew% beträgt. Die Gesamtmenge an Wasser und Alkohol soll jedoch 85 % der stöchiometrischen Menge an Ester und Ether nicht überschreiten.

Solche Reaktionsmischungen, die neben Essigsäure und reaktiven Jodverbindungen auch noch Wasser enthalten, sind jedoch hochkorrosiv gegenüber den meisten technischen Materialien, auch gegenüber Hastelloy-Edelstählen, so daß auf teurere Werkstoffe wie z.B. Tantal zurückgegriffen werden muß.

Überraschenderweise ermöglicht es die vorliegende Erfindung, die beschriebenen Nachteile zu vermeiden. Das Verfahren der Erfindung ist dadurch gekennzeichnet, daß man
a) Methanol und Methylacetat im Molverhältnis 10 : 1 bis 1 : 10 unter wasserfreien Bedingungen mit Kohlenmonoxid oder Gemischen aus Kohlenmonoxid und Wasserstoff in Gegenwart eines Carbonylkomplexes von Edelmetallen der Gruppe VIII des Periodensystems der Elemente, Methyljodid und als Promotor ein Alkali-, quaternäres Organophosphonium- oder Organoammoniumacetat oder ―jodid enthaltenden Katalysatorsystems in einer Reaktionszone bei Temperaturen von 150 bis 250°C und Drücken von 5 bis 120 bar umsetzt,
b) das mit einer Temperatur von 150 bis 250°C die Reaktionszone verlassende Carbonylierungsgemisch in einer Dampf-Flüssigkeits-Abscheidezone auf einen Druck von 1 bis 3,5 bar entspannt, wobei die Hauptmenge der flüchtigen Anteile sofort verdampft und zur Verhinderung des Mitreißens von Flüssigkeitstropfen über einen Nebelabscheider einer ersten Destillationszone zur Abtrennung der Leichtsieder zugeführt wird; aus dem in der Dampf-Flüssigkeitsabscheidezone und im Nebelabscheider anfallenden Flüssigkeitsstrom in einer Trennzone bei einem Druck von 0,05 bis 1 bar und einer Sumpftemperatur von 50 bis 170°C den überwiegenden Teil der noch flüchtigen Anteile abdestilliert und ebenfalls der ersten Destillationszone zuleitet und die als Sumpfprodukt verbleibende Katalysatorlösung zur Reaktionszone zurückführt,
c) bei der fraktionierten Destillation in der ersten Destillationszone über Kopf ein überwiegend aus CO₂, CO und CH₄ und N₂ bestehendes Abgas abzieht, durch Auswaschen mit der Gesamtmenge der Einsatzstoffe Methanol und Methylacetat von restlichem Methyljodid befreit und einer Verbrennung zuleitet und das Gemisch aus Methanol und Methylacetat der Reaktionszone zuführt,
d) in der ersten Destillationszone die flüchtigen Carbonylierungsprodukte durch fraktionierte Destillation unter Normaldruck in einen niedrig siedenden Anteil aus überwiegend Methyljodid und Methylacetat zerlegt und in die Reaktionszone zurückführt und als Sumpfprodukt ein Gernisch aus Essigsäure und Essigsäureanhydrid erhält,
e) das Gemisch von Essigsäure und Essigsäureanhydrid zur Entfernung von Spuren jodhaltiger Verbindungen über einen silbersalzhaltigen Träger leitet oder mit Peressigsäure behandelt und in einer zweiten und dritten Destillationszone durch fraktionierte Destillation in die reinen Komponenten Essigsäure und Essigsäureanhydrid zerlegt,
f) die Verweilzeiten der Einsatzstoffe in der Reaktionszone in Abhängigkeit von den Mengenströmen der zur Reaktionszone zurückgeführten Katalysatorlösung und der Leichtsieder Methyljodid und Methylacetat sowie der Einsatzprodukte Methanol und Methylacetat zwischen 2 bis 50 min. wählt.

Darüber hinaus kann das Verfahren der Erfindung wahlweise und bevorzugt dadurch gekennzeichnet sein, daß
1) das Katalysatorsystem als zusätzlichen Promotor eine Verbindung eines carbonylbildenden Nichtedelmetalls der Gruppen IV bis VIII des Periodensystems der Elemente enthält;
2) man den Einsatzstoff Methylacetat (MA) ganz oder teilweise durch Dimethylether (DME) ersetzt;
3) in dem der Reaktionszone zugeführten Gesamtmengenstrom ein Molverhältnis Edelmetall zu Promotor zu Methyljodid zu Methylacetat von 1 : 2-100 : 10-300 : 10-1000 bei einer Edelmetallkonzentration von 0,005 bis 0,05 mol/l aufrechterhalten wird;
4) das die Reaktionszone verlassende Carbonylierungsgemisch zur Umsetzung gelösten Kohlenmonoxids einen Nachreaktor bei 150 bis 250°C und Verweilzeiten von 0,5 bis 15 min. durchströmt;
5) man die destillative Abtrennung der flüchtigen Anteile von der Katalysatorlösung in der Trennzone in Gegenwart von Kohlenmonoxid oder Gemischen aus Kohlenmonoxid und Wasserstoff durchführt;
6) die Gemische aus Kohlenmonoxid und Wasserstoff bis zu 5 Vol% Wasserstoff enthalten;
7) der Nebelabscheider Filtergewebe aus korrosionsfesten Stoffen, vorzugsweise aus Glasfasern oder Edelstahl, enthält.

Das zur Reaktion eingesetzte Kohlenmonoxid muß nicht unbedingt rein sein. Kleinere Mengen an Inertgasen wie Kohlendioxid, Stickstoff oder Methan stören die Carbonylierung nicht, wenn der Kohlenmonoxid-Partialdruck im Reaktor konstantgehalten wird. Ein Wasserstoffgehalt bis zu 5 Vol% wirkt sich positiv auf die Katalysatoraktivität aus, verringert jedoch die Selektivität des Verfahrens durch Bildung von Hydrierungsprodukten wie z.B. Ethylidendiacetat oder Ethylenglykoldiacetat.

Als Katalysator kann jedes Edelmetall der Gruppe VIII des Periodensystems (Ru, Rh, Pd, Os, Ir, Pt) eingesetzt werden. Die höchste Aktivität besitzt jedoch das Rhodium. Als Einsatzform des Rhodiums können alle Verbindungen dienen, die unter den Reaktionsbedingungen löslich sind und den aktiven Edelmetallcarbonylkomplex bilden, z.B. Rhodiumchlorid, Rh-aceatet, Rhodiumcarbonylchlorid.

Von den als Promotorsalz eingesetzten Alkalijodiden kommt dem Lithiumjodid die größte Bedeutung zu, doch können auch Natriumjodid oder Kaliumjodid verwendet werden. Als quaternäres Organophosphoniumjodid wird bevorzugt Methyltributylphosphoniumjodid eingesetzt, jedoch ist auch der Einsatz anderer Phosphoniumjodide wie Methyltriphenylphosphoniumjodid, Tetrabutylphosphoniumjodid oder Dimethyldibutylphosphoniumjodid möglich. Als quaternäre Organoammoniumverbindung wird bevorzugt das N,N-Dimethylimidazoliumjodid eingesetzt, jedoch sind auch N-Methylpyridiniumjodid, N-Methyl-3-picoliniumjodid, N-Methyl-2,4-lutidiniumjodid, N-Methyl-3,4-lutidiniumjodid, N-Methylchinoliniumjodid und andere verwendbar. Die Konzentration des Promotorsalzes in der Reaktionsmischung kann zwischen 0,01 und 5 mol/l, vorteilhaft zwischen 0,1 und 1 mol/l liegen.

Die gegebenenfalls als Copromotoren verwendeten und Carbonylkomplexe bildenden Nichtedelmetalle der Gruppen IV, V, VI, VII und VIII des Periodensystems der Elemente werden zweckmäßig in einer gut löslichen Form, z.B. als Acetylacetonat oder Carbonyl in die Reaktion eingesetzt. Die Konzentrationen dieser Copromotoren in der Reaktionsmischung liegen zweckmäßig bei 0,01 bis 0,5 mol/l, bevorzugt bei 0,05 bis 0,3 mol/l. Es kommen hier vorzugsweise Verbindungen der Metalle Ti, Zr, V, Nb, Ta, Cr, Mo, W, Mn, Re, Fe, Co oder Ni infrage.

Ein besonderer Vorteil des Verfahrens der Erfindung liegt darin, daß sich durch Variation im Verhältnis der Einsatzprodukte Methanol und Methylacetat praktisch jedes Produktverhältnis von Essigsäure und Essigsäureanhydrid einstellen läßt, so daß das Verfahren schnell an wechselnde Erfordernisse angepaßt werden kann.

Das Verfahren arbeitet bevorzugt in flüssiger Phase bei Betriebsdrucken zwischen 20 und 80 bar. Das Carbonylierungsverfahren kann sowohl in einer diskontinuierlichen als auch in einer kontinuierlichen Anlage betrieben werden.

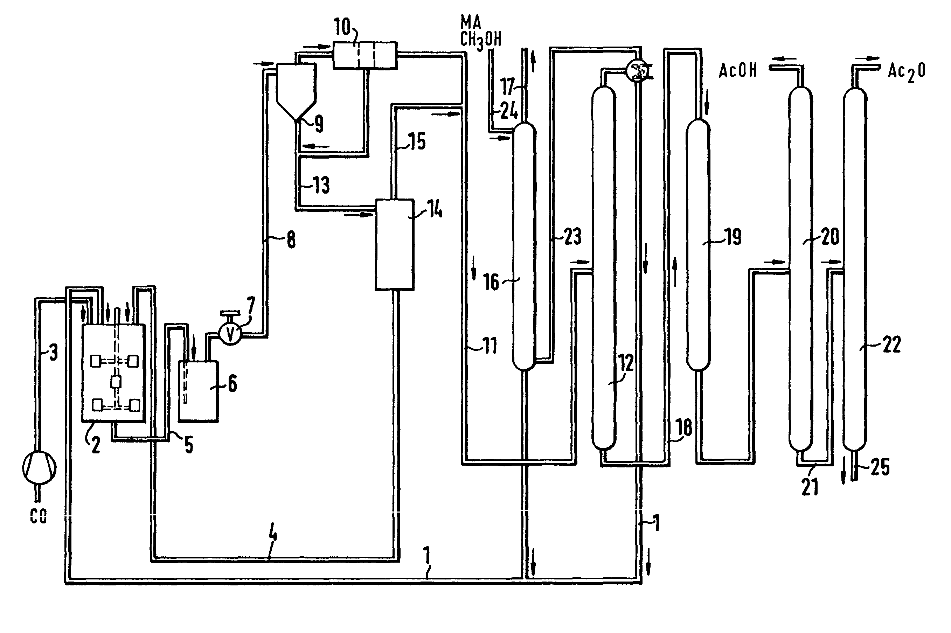

Die Erfindung wird im folgenden anhand der Zeichnung näher erläutert:
Die Einsatzstoffe Methylacetat (MA) und Methanol werden über Leitung 24, Abgaswäsche 16 und Leitung 1 gemeinsam mit den im Kreislauf geführten Leichtsiedern Methyljodid und Methylacetat dem Reaktor 2 zugeführt. Über Leitung 3 werden Kohlenmonoxid und über Leitung 4 die im Kreislauf geführte Katalysatorlösung ebenfalls dem Reaktor zugeführt. Unter Einhaltung eines konstanten Reaktionsvolumens werden nach Maßgabe der Zuführung der Einsatzstoffe die Reaktionsprodukte über Leitung 5 in den Nachreaktor 6 abgezogen und über das Entspannungsventil 7 und die Leitung 8 in den Dampf-Flüssigkeitsabscheider 9 entspannt. Die Temperatur der Einsatzstoffe ist dabei so gewählt, daß die Reaktionswärme durch Aufheizen der Einsatzstoffe auf die Reaktionstemperatur von 150 bis 250°C abgeführt werden kann. Im Dampf-Flüssigkeitsabscheider 9 wird unter Entspannung und Ausnutzung des Wärmeinhaltes der überwiegende Anteil der Leichtsieder verdampft. Der Dampfstrom wird nach Abscheidung mitgerissener Flüssigkeitströpfchen im Nebelabscheider 10 über die Leitung 11 der Leichtsiederkolonne 12 zugeführt. Der im Dampf-Flüssigkeitsabscheider 9 und im Nebelabscheider 10 anfallende Flüssigkeitsstrom wird über Leitung 13 der Trennstufe 14 zugeführt. Hier werden weitere verdampfbare Anteile abgetrennt und über Leitung 15 und 11 ebenfalls der Leichtsiederkolonne 12 zugeführt, während die in der Trennstufe 14 als Sumpfprodukt anfallende Katalysatorlösung über Leitung 4 in den Reaktor 2 zurückgelangt. In der Leichtsiederkolonne 12 werden unter Normaldruck die Leichtsieder Methyljodid und nicht umgesetztes Methylacetat über Kopf abgetrennt und über Leitung 1 in den Reaktor 2 zurückgeleitet. Über den Kondensator der Leichtsiederkolonne 12 wird das Abgas über die Leitung 23 entnommen, in der Abgaswäsche 16 mit der Gesamtmenge der Einsatzstoffe Methylacetat und Methanol gewaschen und so von noch mitgeführtem Methyljodid befreit. Das Abgas (CO₂, CO, CH₄, N₂) verläßt die Anlage über Leitung 17. Das Sumpfprodukt der Leichtsiederkolonne 12 besteht überwiegend aus Essigsäure und Essigsäureanhydrid und wird über Leitung 18 in die mit einem silbersalzhaltigen Träger, z.B. lonenaustauscherharz, gefüllte Kolonne 19 geleitet, wo die in den beiden Produkten noch in Spuren vorhandenen Jodverbindungen entfernt werden. Dies kann an gleicher Stelle ohne silbersalzhaltige Träger auch durch Behandlung der beiden Produkte mit Peressigsäure erfolgen. Die jodfreien Produkte werden in der Kolonne 20 unter vermindertem Druck fraktioniert. Als Kopfprodukt der Kolonne 20 wird reine Essigsäure erhalten. Das Sumpfprodukt wird über Leitung 21 der Kolonne 22 zugeführt, in der über Kopf unter vermindertem Druck reines Essigsäureanhydrid anfällt, während die Hochsieder als Sumpfprodukt über Leitung 25 abgezogen werden.

### Beispiel 1

Die Carbonylierung erfolgt bei einer Temperatur von 190°C unter einem Gesamtdruck von 50 bar. Das genutzte Reaktorvolumen beträgt 3 Liter. Das Reaktionsgemisch enthält den Rhodiumkomplex, Methyltributylphosphoniumjodid, Methyljodid und Methylacetat im Molverhältnis 1 : 18 : 80 : 170. Die Edelmetallkonzentration beläuft sich auf 26,5 mmolRh/l Reaktionsgemisch.

Stündlich werden 0,95 kg Methylacetat (12,8 mol) und 1,9 kg Methanol (59,4 mol) durch Leitung 24 über die Abgaswäsche 16 sowie durch Leitung 1 dem Reaktor 2 zugeführt. Durch Leitung 3 strömen gleichzeitig 2 kg Kohlenmonoxid (72,4 mol) in den Reaktor. Ferner werden über Leitung 4 stündlich 4,6 kg Katalysatorlösung und über Leitung 1 im Kreislauf 11,8 kg Leichtsieder (Methyljodid, Methylacetat) in den Reaktor eingeleitet. Über die Leitung 5 werden dem Reaktor 2 stündlich 21,25 kg (≙ 18,6 l) entnommen, woraus sich eine mittlere Verweilzeit im Reaktor von annähernd 10 min ergibt. Das im Reaktionsgemisch noch gelöste CO wird im Nachreaktor 6 (1 l Volumen) unter Reaktionsdruck bei 185°C nahezu vollständig umgesetzt. Die Verweilzeit beträgt hier etwas mehr als 3 min. Im Maße der Aufgabe der Produkte in den Reaktor 2 werden unter Standhaltung die Reaktionsprodukte über das Entspannungsventil 7 auf 1 bar entspannt und gelangen über Leitung 8 in den Dampf-Flüssigkeitsabscheider 9, in dem sich eine mittlere Temperatur von 95 °C einstellt. Dampfförmig strömen 11,2 kg/h über den mit Glasfasern ausgestatteten Nebelabscheider 10, in dem bei 90°C mitgerissene katalysatorhaltige Flüssigkeitströpfchen abgeschieden werden, durch die Leitung 11 in die Leichtsiederkolonne 12. Der flüssige Anteil aus Abscheider 9 und Nebelabscheider 10 gelangt über Leitung 13 in die Trennstufe 14, wo unter Normaldruck und 145°C weitere 5,45 kg/h verdampft und durch die Leitungen 15 und 11 ebenfalls der Leichtsiederkolonne 12 zugeführt werden. Die in der Trennstufe 14 flüssig abgetrennte katalysatorhaltige Lösung wird über Leitung 4 dem Reaktor 2 erneut aufgegeben.

In der Leichtsiederkolonne 12 erfolgt unter Normaldruck bei 126°C Sumpf- und 70°C Kopftemperatur die Abtrennung der Leichtsieder Methyljodid und Methlyacetat, die über Leitung 1 im Kreislauf erneut in die Reaktion eingesetzt werden. Das aus CO, CO₂, CH₄ und Inerten (N₂) bestehende Abgas wird über Leitung 23 abgezogen und in der Abgaswäsche 16 bei 0°C im Gegenstrom mit den Einsatzprodukten Methanol/Methylacetat von partialdruckmäßig mitgeführtem Methyljodid befreit. Das Abgas (0,05 kg/h) wird über Leitung 17 der Verbrennung zugeführt.

Das aus Essigsäure, Essigsäureanhydrid und Hochsiedern bestehende Sumpfprodukt der Leichtsiederkolonne 12 (4,8 kg/h) gelangt über Leitung 18 in die mit einem silberhaltigen lonenaustauscher gefüllte Kolonne 19 und wird dort bei 50°C und einer Verweilzeit von 30 min von Spuren noch enthaltener Jodverbindungen gereinigt.

Anschließend erfolgt in der Kolonne 20 unter einem Druck von 150 mbar bei 70°C Kopf- und 99°C Sumpftemperatur die Abtrennung von 3,5 kg/h reiner Essigsäure (58,3 mol). Dies entspricht einer Ausbeute, bezogen auf das eingesetzte Methanol, von 98,1 %.

Der Sumpf der Kolonne 20 wird in der Kolonne 22 unter ebenfalls 150 mbar Druck fraktioniert. Bei 90°C Kopf- und 104°C Sumpftemperatur fallen 1,25 kg/h reines Essigsäureanhydrid (12,25 mol) an, was einer Ausbeute von 95,7 %, bezogen auf das umgesetzte Methylacetat, entspricht. Als Sumpfprodukt der Kolonne 22 werden 0,05 kg/h Hochsieder über die Leitung 25 abgezogen. Die Ausbeute an Essigsäure und Essigsäureanhydrid, bezogen auf den CO-Einsatz, beträgt 97,4 %. Die Reaktorleistung liegt bei 1583 g Essigsäure und Essigsäureanhydrid pro Liter Reaktionsvolumen und Stunde. Die Carbonylierungsleistung beläuft sich auf 667 g CO/l Rv • h.

### Beispiel 2

Bei gegenüber Beispiel 1 unveränderten Reaktionsbedingungen beträgt das genutzte Reaktorvolumen 4,5 Liter. Das Reaktionsgemisch enthält den Rhodiumkomplex, Methyltributylphosphoniumjodid, Methyljodid und Dimethylether im Molverhältnis 1 : 18 : 78 : 152. Die Edelmetallkonzentration beläuft sich auf 27 mmol Rhodium/l Reaktionsgemisch. Stündlich werden 1,5 kg Dimethylether (32,4 mol) und 0,7 kg Methanol (22,5 mol) durch Leitung 1 dem Reaktor 2 zugeführt. Durch Leitung 3 strömen in der gleichen Zeit 2,5 kg CO (88 mol) in den Reaktor. Ferner werden über Leitung 4 stündlich 7,1 kg Katalysatorlösung und über Leitung 1 im Kreislauf 21,2 kg Leichtsieder (Methyljodid, Methylacetat) in den Reaktor eingeleitet. Über die Leitung 5 werden dem Reaktor 2 stündlich 33 kg (≙ 28,7 l) entnommen, woraus sich eine Verweilzeit im Reaktor von 9,4 min ergibt. Das im Reaktionsgemisch noch gelöste CO wird im Nachreaktor 6 mit einer Verweilzeit von 2 min unter Reaktionsdruck bei 185°C nahezu vollständig umgesetzt. Im Maße der Aufgabe der Produkte in den Reaktor 2 werden unter Standhaltung die Reaktionsprodukte über das Entspannungsventil 7 auf 1 bar entspannt und gelangen über Leitung 8 in den Dampf-Flüssigkeitsabscheider 9, in dem sich eine mittlere Temperatur von 105°C einstellt. Dampfförmig strömen 17,3 kg/h über den mit Glasfasern ausgestatteten Nebelabscheider 10, in dem bei 95°C mitgerissene katalysatorhaltige Flüssigkeitströpfchen abgeschieden werden, durch die Leitung 11 in die Leichtsiederkolonne 12. Der flüssige Anteil aus Abscheider 9 und Nebelabscheider 10 gelangt über Leitung 13 in die Trennstufe 14, in der unter einem Druck von 150 mbar und 95°C unter Zudosierung von 20 l/h CO mit 5 Vol.% Wasserstoff weitere 8,6 kg/h verdampft und durch Leitungen 15 und 11 ebenfalls der Leichtsiederkolonne 12 zugeführt werden. Die in der Trennstufe 14 flüssig abgetrennte katalysatorhaltige Lösung wird über Leitung 4 dem Reaktor 2 erneut aufgegeben.

In der Leichtsiederkolonne 12 erfolgt unter Normaldruck bei 132°C Sumpf- und 78°C Kopftemperatur die Abtrennung der Leichtsieder Methyljodid und Methylacetat, die über Leitung 1 im Kreislauf erneut in die Reaktion eingesetzt werden. Das aus CO, CO₂, Methan und Inerten (N₂) bestehende Abgas wird über die Leitung 23 abgezogen und in der Abgaswäsche 16 bei -20°C im Gegenstrom mit dem Einsatzprodukt Methanol von partialdruckmäßig mitgeführtem Methyljodid befreit. Das Abgas (0,05 kg/h) wird über Leitung 17 der Verbrennung zugeführt. Das aus Essigsäure, Essigsäureanhydrid und Hochsiedern bestehende Sumpfprodukt der Leichtsiederkolonne 12 (4,65 kg/h) gelangt über die Leitung 18 in die Kolonne 19 und wird dort bei 120°C und einer mittleren Verweilzeit von 20 min durch Zusatz von 140 g/h einer 10 %igen Lösung von Peressigsäure in Essigsäure behandelt. Anschließend erfolgt in der Kolonne 20 unter einem Druck von 150 mbar bei 70°C Kopf- und 99°C Sumpftemperatur die Abtrennung von 1,32 kg/h reiner Essigsäure (22 mol). Dies entspricht einer Ausbeute, bezogen auf das eingesetzte Methanol, von 97,8 %. Der Sumpf der Kolonne 20 wird in der Kolonne 22 unter ebenfalls 150 mbar Druck fraktioniert. Bei 90°C Kopf- und 104°C Sumpftemperatur fallen 3,28 kg/h reines Essigsäureanhydrid (32,15 mol) an, was einer Ausbeute von 99,2 %, bezogen auf den eingesetzten Dimethylether, entspricht. Als Sumpfprodukt der Kolonne 22 werden 0,05 kg/h Hochsieder über die Leitung 25 entfernt. Die Ausbeute an Essigsäure und Essigsäureanhydrid, bezogen auf den CO-Einsatz, beträgt 98 %. Die Reaktorleistung liegt bei 1022 g Essigsäure und Essigsäureanhydrid/l Rv • h. Die Carbonylierungsleistung beläuft sich auf 556 g CO/l Rv • h.

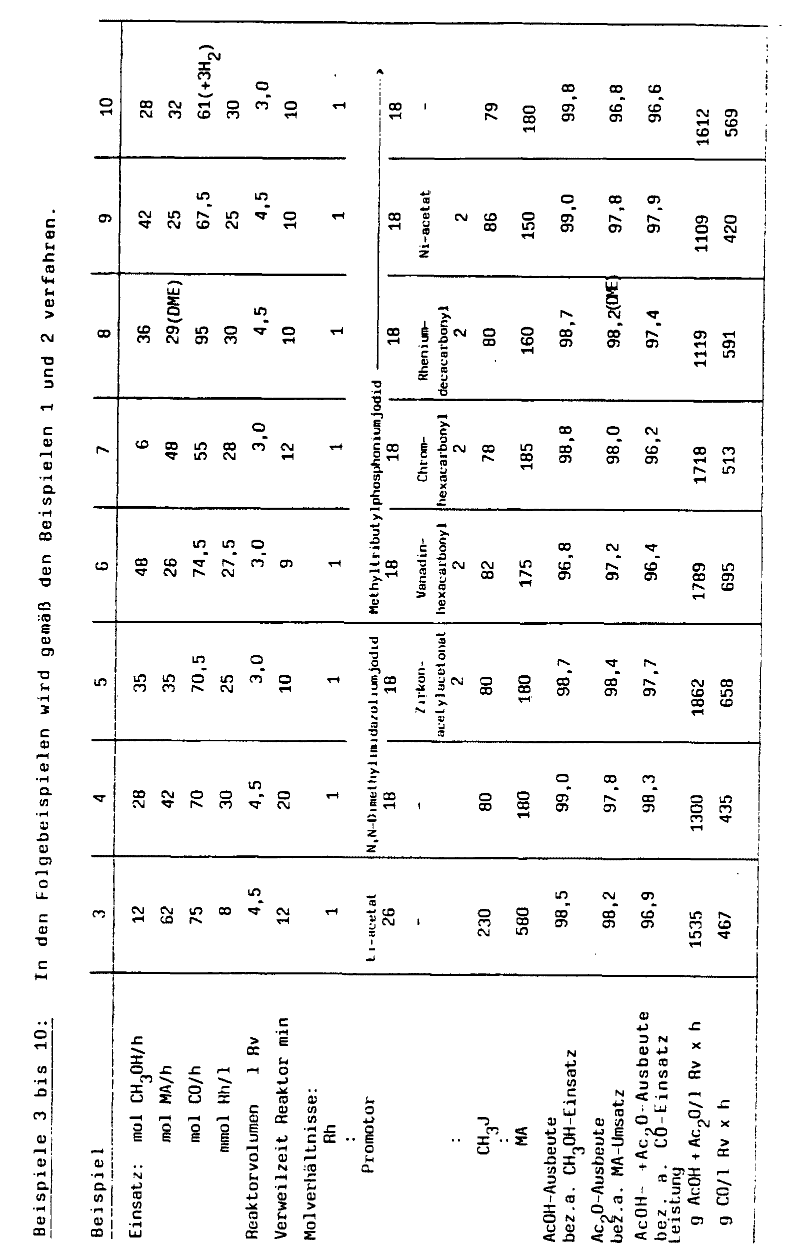

## Patentansprüche

1. Verfahren zur Herstellung von Essigsäure und Essigsäureanhydrid durch Umsetzung von Methanol und Methylacetat mit Kohlenmonoxid, **dadurch gekennzeichnet, daß** man
a) Methanol und Methylacetat im Molverhältnis 10 : 1 bis 1 : 10 unter wasserfreien Bedingungen mit Kohlenmonoxid oder Gemischen aus Kohlenmonoxid und Wasserstoff in Gegenwart eines Carbonylkomplexes von Edelmetallen der Gruppe VIII des Periodensystems der Elemente, Methyljodid und als Promotor ein Alkali-, quaternäres Organophosphonium- oder Organoammoniumacetat oder ―jodid enthaltenden Katalysatorsystems in einer Reaktionszone bei Temperaturen von 150 bis 250°C und Drücken von 5 bis 120 bar umsetzt,
b) das mit einer Temperatur von 150 bis 250°C die Reaktionszone verlassende Carbonylierungsgemisch in einer Dampf-Flüssigkeits-Abscheidezone auf einen Druck von 1 bis 3,5 bar entspannt, wobei die Hauptmenge der flüchtigen Anteile sofort verdampft und zur Verhinderung des Mitreißens von Flüssigkeitstropfen über einen Nebelabscheider einer ersten Destillationszone zur Abtrennung der Leichtsieder zugeführt wird; aus dem in der Dampf-Flüssigkeitsabscheidezone und im Nebelabscheider anfallenden Flüssigkeitsstrom in einer Trennzone bei einem Druck von 0,05 bis 1 bar und einer Sumpftemperatur von 50 bis 170°C den überwiegenden Teil der noch flüchtigen Anteile abdestilliert und ebenfalls der ersten Destillationszone zuleitet und die als Sumpfprodukt verbleibende Katalysatorlösung zur Reaktionszone zurückführt,
c) bei der fraktionierten Destillation in der ersten Destillationszone über Kopf ein überwiegend aus CO₂, CO und CH₄ und N₂ bestehendes Abgas abzieht, durch Auswaschen mit der Gesamtmenge der Einsatzstoffe Methanol und Methylacetat von restlichem Methyljodid befreit und einer Verbrennung zuleitet und das Gemisch aus Methanol und Methylacetat der Reaktionszone zuführt,
d) in der ersten Destillationszone die flüchtigen Carbonylierungsprodukte durch fraktionierte Destillation unter Normaldruck in einen niedrig siedenden Anteil aus überwiegend Methyljodid und Methylacetat zerlegt und in die Reaktionszone zurückführt und als Sumpfprodukt ein Gernisch aus Essigsäure und Essigsäureanhydrid erhält,
e) das Gemisch von Essigsäure und Essigsäureanhydrid zur Entfernung von Spuren jodhaltiger Verbindungen über einen silbersalzhaltigen Träger leitet oder mit Peressigsäure behandelt und in einer zweiten und dritten Destillationszone durch fraktionierte Destillation in die reinen Komponenten Essigsäure und Essigsäureanhydrid zerlegt,
f) die Verweilzeiten der Einsatzstoffe in der Reaktionszone in Abhängigkeit von den Mengenströmen der zur Reaktionszone zurückgeführten Katalysatorlösung und der Leichtsieder Methyljodid und Methylacetat sowie der Einsatzprodukte Methanol und Methylacetat zwischen 2 bis 50 min. wählt.

2. Verfahren nach Anspruch 1, **dadurch gekennzeichnet, daß** das Katalysatorsystem als zusätzlichen Promotor eine Verbindung eines carbonylbildenden Nichtedelmetalls der Gruppen IV bis VIII des Periodensystems der Elemente enthält.

3. Verfahren nach einem der vorhergehenden Ansprüche, **dadurch gekennzeichnet, daß** man den Einsatzstoff Methylacetat ganz oder teilweise durch Dimethylether ersetzt.

4. Verfahren nach einem der vorhergehenden Ansprüche, **dadurch gekennzeichnet, daß** in dem der Reaktionszone zugeführten Gesamtmengenstrom ein Molverhältnis Edelmetall zu Promotor zu Methyljodid zu Methylacetat von 1 : (2 bis 100) : (10 bis 300) : (10 bis 1000) bei einer Edelmetallkonzentration von 0,005 bis 0,05 mol/l aufrechterhalten wird.

5. Verfahren nach einem der vorhergehenden Ansprüche, **dadurch gekennzeichnet, daß** das die Reaktionszone verlassende Carbonylierungsgemisch zur Umsetzung gelösten Kohlenmonoxids einen Nachreaktor bei 150 bis 250°C und Verweilzeiten von 0,5 bis 15 min. durchströmt.

6. Verfahren nach einem oder vorhergehenden Ansprüche, **dadurch gekennzeichnet, daß** man die destillative Abtrennung der flüchtigen Anteile von der Katalysatorlösung in der Trennzone in Gegenwart von Kohlenmonoxid oder Gemischen aus Kohlenmonoxid und Wasserstoff durchführt.

7. Verfahren nach einem der vorhergehenden Ansprüche, **dadurch gekennzeichnet, daß** die Gemische aus Kohlenmonoxid und Wasserstoff bis zu 5 Vol.% Wasserstoff enthalten.

8. Verfahren nach einem der vorhergehenden Ansprüche, **dadurch gekennzeichnet, daß** der Nebelabscheider Filtergewebe aus korrosionsfesten Stoffen, vorzugsweise aus Glasfasern oder Edelstahl, enthält.

## Claims

1. A process for the preparation of acetic acid and acetic anhydride by reacting methanol and methyl acetate with carbon monoxide, wherein
a) methanol and methyl acetate in the molar ratio 10 : 1 to 1 : 10 are reacted under anhydrous conditions with carbon monoxide or mixtures of carbon monoxide and hydrogen in the presence of a carbonyl complex of noble metals from group VIII of the Periodic Table of the Elements, methyl iodide and, as promoter, a catalyst system containing an acetate or iodide of an alkali metal, quaternary organophosphonium or organoammonium, in a reaction zone at temperatures of from 150 to 250°C and pressures of from 5 to 120 bar;
b) the carbonylation mixture leaving the reaction zone at a temperature of from 150 to 250°C is decompressed to a pressure of from 1 to 3.5 bar in a vapor-liquid deposition zone, the major part of the volatile components evaporating immediately and, in order to prevent entrainment of liquid drops, being fed via a mist eliminator to a first distillatione zone for removal of the low-boiling components; the major part of the still-volatile components is distilled off in a separation zone at a pressure of from 0.05 to 1 bar and a bottom temperature of from 50 to 170°C from the liquid stream produced in the vapor-liquid deposition zone and in the mist eliminator and is likewise fed to the first distillation zone, and the catalyst solution which remains as the bottom product is fed back to the reaction zone;
c) during fractional distillation in the first distillation zone, an offgas, predominantly compising CO₂, CO, CH₄ and N₂, is withdrawn at the head of the column, freed from residual methyl iodide by washing with the total amount of the starting materials methanol and methyl acetate, and passed to combustion, and the mixture of methanol and methyl acetate is fed to the reaction zone;
d) the volatile carbonylation products are split in the first distillation zone by fractional distillation under atmospheric pressure into a low-boiling component predominantly comprising methyl iodide and methyl acetate, and are fed back into the reaction zone, and the bottom product obtained is a mixture of acetic acid and acetic anhydride;
e) in order to remove traces of iodine-containing compounds, the mixture of acetic acid and acetic anhydride is passed over a carrier containing a silver salt or treated with peracetic acid, and is split into the pure components, acetic acid and acetic anhydride, by fractional distillation in a second distillation zone and a third distillation zone;
f) the residence time of the starting materials in the reaction zone are from 2 to 50 minutes, depending on the flow rates of the catalyst solution fed back to the reaction zone and of the low-boiling components methyl iodide and methyl acetate and of the starting materials methanol and methyl acetate.

2. A process as claimed in claim 1, wherein the catalyst system contains, as additional promoter, a compound of a carbonyl-forming non-noble metal from groups IV to VIII of the Periodic Table of the Elements.

3. A process as claimed in any one of the preceding claims, wherein the starting material methyl acetate is replaced fully or partly by dimethyl ether.

4. A process as claimed in any one of the preceding claims, wherein a noble metal : promoter: methyl iodide : methyl acetate molar ratio of 1 : 2 ― 100 : 10 ― 300 : 10 ― 1000 is maintained in the overall stream fed to the reaction zone at a noble metal concentration of from 0.005 to 0.05 mol/l.

5. A process as claimed in any one of the preceding claims, wherein the carbonylation mixture leaving the reaction zone flows through a subsequent reactor at 150 to 250°C and residence times of from 0.5 to 15 minutes in order to convert dissolved carbon monoxide.

6. A process as claimed in any one of the preceding claims, wherein the distillative separation of the volatile components from the catalyst solution in the separation zone is carried out in the presence of carbon monoxide or mixtures of carbon monoxide and hydrogen.

7. A process as claimed in any one of the preceding claims, wherein the mixtures of carbon monoxide and hydrogen contain up to 5 % by volume of hydrogen.

8. A process as claimed in any one of the preceding claims, wherein the mist eliminator contains filter fabrics made from corrosion-resistant substances, preferably glass fibers or stainless steel.

## Revendications

1. Procédé pour la préparation de l'acide acétique et de l'anhydride acétique par réaction du méthanol et de l'acétate de méthyle avec l'oxyde de carbone, **caractérisé en ce que**
a) on fait réagir dans une zone de réaction, à des températures de 150 à 250°C et des pressions de 5 à 120 bar, le méthanol et l'acétate de méthyle dans un rapport molaire de 10:1 à 1:10 en milieu anhydre avec l'oxyde de carbone ou des mélanges d'oxyde de carbone et d'hydrogène, en présence d'un système catalyseur contenant des complexes carbonylés de métaux nobles du groupe VIII de la Classification Périodique des Eléments, de l'iodure de méthyle, et, en tant qu'activateur, un acétate ou iodure alcalin, d'organo-phosphonium quaternaire ou d'organo-ammonium quaternaire,
b) on détend le mélange de carbonylation quittant la zone de réaction à une température de 150 à 250°C à une pression de 1 à 3,5 bar dans une zone de séparation vapeurs-liquide, ce qui provoque la vaporisation immédiate de la plus grande partie des fractions volatiles qu'on envoie, en passant par un séparateur à brouillards servant à éviter l'entraînement de gouttelettes de liquide, à une première zone de distillation pour séparation des fractions volatiles ; à partir du courant de liquide provenant de la zone de séparation vapeurs-liquide et du séparateur à brouillards, on distille dans une zone de séparation, à une pression de 0,05 à 1 bar et à une température de liquide de 50 à 170°C, la plus grande partie des fractions encore volatiles qu'on envoie également à la première zone de distillation, et on renvoie à la zone de réaction la solution de catalyseur restant en produit de culot,
c) à la distillation fractionnée dans la première zone de distillation, on évacue en tête des gaz résiduaires consistant principalement en CO₂, CO, CH₄ et N₂ qu'on débarrasse des résidus d'iodure de méthyle par lavage avec la totalité des matières premières mises en oeuvre : méthanol et acétate de méthyle, et qu'on envoie à la combustion, et on recycle le mélange de méthanol et d'acétate de méthyle à la zone de réaction,
d) on fractionne les produits de carbonylation volatils dans la première zone de distillation par distillation fractionnée à pression normale en une fraction à bas point d'ébullition consistant principalement en iodure de méthyle et acétate de méthyle qu'on recycle à la zone de réaction, et on recueille en produit de culot un mélange d'acide acétique et d'anhydride acétique,
e) on envoie le mélange d'acide acétique et d'anhydride acétique, pour élimination de traces de dérivés iodés, sur un support contenant un sel d'argent ou bien on le traite par l'acide peracétique et on le fractionne dans une deuxième et une troisième zone de distillation, par distillation fractionnée, en les composants purs : acide acétique et anhydride acétique,
f) les durées de passage des produits mis en oeuvre dans la zone de réaction, selon les débits des courants de la solution de catalyseur recyclée à la zone de réaction, des produits à bas point d'ébullition : iodure de méthyle et acétate de méthyle, et des produits mis en oeuvre : méthanol et acétate de méthyle, sont de 2 à 50 min.

2. Procédé selon la revendication 1, **caractérisé en ce que** le système catalyseur contient, en tant qu'activateur supplémentaire, un composé d'un métal non noble des groupes IV à VIII de la Classification Périodique des Eléments, formant des dérivés carbonylés.

3. Procédé selon une des revendications qui précèdent, **caractérisé en ce que** l'on remplace l'acétate de méthyle mis en oeuvre, en totalité ou en partie, par de l'éther diméthylique.

4. Procédé selon une des revendications qui précèdent, **caractérisé en ce que**, dans le courant de produits total envoyé à la zone de réaction, on maintient des proportions molaires métal noble/activateur/iodure de méthyle/acétate de méthyle de 1:2 à 100:10 à 300:10 à 1 000 à une concentration en métal noble de 0,005 à 0,05 mol/h.

5. Procédé selon une des revendications qui précèdent, **caractérisé en ce que** le mélange de carbonylation quittant la zone de réaction passe dans un réacteur complémentaire à des températures de 150 à 250°C avec des durées de passage de 0,5 à 15 min, pour conversion de l'oxyde de carbone dissous.

6. Procédé selon une des revendications qui précèdent, **caractérisé en ce que** l'on procède à la séparation par distillation des fractions volatiles et de la solution de catalyseur dans la zone de séparation en présence d'oxyde de carbone ou de mélanges d'oxyde de carbone et d'hydrogène.

7. Procédé selon une des revendications qui précèdent, **caractérisé en ce que** les mélanges d'oxyde de carbone et d'hydrogène contiennent jusqu'à 5 % en volume d'hydrogène.

8. Procédé selon une des revendications qui précèdent, **caractérisé en ce que** le séparateur à brouillards contient des tissus filtrants en étoffe résistant à la corrosion, de préférence en fibres de verre ou en acier inoxydable.
